(19) Europäisches Patentamt

European Patent Office

Office européen des brevets

(11) **EP 3 062 093 B1**

(12) **EUROPEAN PATENT SPECIFICATION**

(45) Date of publication and mention
of the grant of the patent:
**26.07.2023   Bulletin 2023/30**

(21) Application number: **14855053.6**

(22) Date of filing: **23.10.2014**

(51) International Patent Classification (IPC):
**G01N 23/083** (2018.01)       **G01T 1/24** (2006.01)
**A61B 6/03** (2006.01)       **A61B 6/00** (2006.01)

(52) Cooperative Patent Classification (CPC):
**G01N 23/083; A61B 6/4241; A61B 6/502;
G01T 1/24;** A61B 6/03; A61B 6/484; A61B 6/52;
G01N 2223/419; G01N 2223/501

(86) International application number:
**PCT/CN2014/089368**

(87) International publication number:
**WO 2015/058702 (30.04.2015 Gazette 2015/17)**

(54) **PHOTON COUNT-BASED RADIATION IMAGING SYSTEM, METHOD, AND APPARATUS**

AUF PHOTONENZÄHLUNG BASIERTES STRAHLUNGSBILDGEBUNGSSYSTEM, VERFAHREN UND VORRICHTUNG

SYSTÈME D'IMAGERIE PAR RAYONNEMENT À COMPTAGE DE PHOTONS, PROCÉDÉ ET APPAREIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **23.10.2013   CN 201310504035
04.04.2014   CN 201410137171
15.07.2014   CN 201410337142**

(43) Date of publication of application:
**31.08.2016   Bulletin 2016/35**

(73) Proprietor: **Nanovision Technology (Beijing) Co.,
Ltd.
Haidian District
Beijing
100094 (CN)**

(72) Inventors:
• **LI, Yunxiang
  Beijing 100085 (CN)**
• **ZHENG, Hailiang
  Beijing 100038 (CN)**
• **CAO, Hongguang
  Haidian District
  Beijing 100089 (CN)**

(74) Representative: **Keil & Schaafhausen
Patentanwälte PartGmbB
Friedrichstraße 2-6
60323 Frankfurt am Main (DE)**

(56) References cited:
**WO-A1-2013/095706        CN-A- 1 589 744
CN-A- 101 214 154        CN-A- 102 099 704
CN-A- 102 221 565        CN-U- 202 522 706
US-A1- 2004 066 904        US-A1- 2006 008 046
US-A1- 2008 101 534**

**Description**

**BACKGROUND**

**Technical Field**

The present invention relates to a radiation imaging system, particularly to a photon count-based X-ray imaging system, and also to a method for achieving X-ray imaging by the system and key devices thereof, which belong to the field of medical image technologies.

**Related Art**

The penetrating power of X-rays is particularly strong. When the X-rays pass through a sample made up of light elements such as carbon, hydrogen and oxygen, they leave no observable traces like visible light penetrates glass. This is very adverse for medical diagnosis. For example, for the diagnosis of breast tumor, the breast tumor, in the early stage of the development, is still the focus composed of light elements, the absorption-contrast imaging is helpless, and it is visible to the absorption-contrast imaging until calcification is generated in the late stage of the development of the breast tumor. This has missed the best time to treatment, seriously affecting the patients' chances of recovery.

With continuous development of the X-ray imaging technology, it is found that phase information carried by the X-rays after penetrating the sample can also be used for imaging the internal structure of the sample, and the phase drift section of the X-rays is 100-1000 times higher than the absorption section; the internal structure of the sample can be observed by acquiring phase information and carrying out recovery. For weak absorption materials composed of light elements, the change of the phase of the X-rays is more evident that the change of light intensity. X-ray phase-contrast imaging can more easily detect the internal structure of the sample than the traditional absorption-contrast imaging.

After more than 30 years of development, the X-ray phase-contrast imaging technology mainly uses the following four methods:

(1) Crystal interference contrast-imaging method: A complete crystal is cut into three very thin 3L shapes with bases connected together, which are respectively a beam splitter, a projection crystal and an analysis crystal. An X-ray is incident and passes through the first crystal, and then is diffracted and separated into two beams of coherent light. One beam of light is used as reference light, and a phase changer is placed on a propagation path thereof to continuously change light. The method is relatively strict in demanding mechanical stability of the experiment device; as a final diffraction pattern is detected after the incident X-ray penetrates 3 layers of crystals, the photon utilization is low, and a strong light source or a longer exposure time is required to make up. As the crystal size is limited, the method is only suitable for some small-size samples, and is only applicable to synchronous radiation at present.

(2) Crystal diffraction enhancement method: after heterogeneous X-rays emitted by the X-ray source go through a complete crystal, X-rays of which the incident angle meets the bragg diffraction condition (that is, the condition of producing coherent light interference) can pass through a monochromatic crystal, thus forming monochromatic light. An analysis crystal is placed behind the sample to serve as an angle analyzer, followed by a detector to record images. After the monochromatic light penetrates the sample, the analysis crystal converts phase information to light intensity information. By using the analysis crystal and adjusting the angle of the analysis crystal, X-rays transmitted, refracted and small-angle scattered after passing through the sample are enhanced or weakened, and thus diffraction enhancement imaging has three mechanisms of producing contrast, which are respectively absorption contrast, refraction contrast and extinction contrast obtained by filtering small-angle scattering.

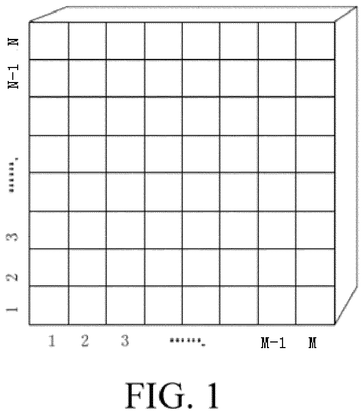

(3) Grating shearing method: the monochromatic light is used to irradiate a grating, and a periodic image may appear at a certain distance behind the grating, that is, the "Tablot-Lau effect", as shown in FIG. 1. By use of the grating self-imaging effect and through design of a light path, an image of a first phase grating is matched with a second absorption grating, then moire fringes formed by the sample are analyzed, and wave fronts can be quantitatively recovered. At present, there are two implementation schemes for the method, one is producing a phase shift of $\pi/2$ and the other is producing a phase shift of $\pi$. The advantage of the method lies in no longer relying on synchrotron radiation light sources with high brightness and higher coherence, thus having an extensive application prospect. The method (2) and the method (3) are X-ray phase-contrast imaging methods based on optical analysis elements. The function of such optical analysis elements is to generate phase differential images, thus improving the boundary contrast of the images, and quantitative phase recovery needs to be carried out through a certain experimental mechanism and a corresponding algorithm.

(4) Phase-contrast imaging method based on X-ray free propagation: the method is also referred to as an X-ray in-line phase-contrast imaging method, which, according to different light sources used, is divided into monochromatic X-ray in-line phase-contrast imaging and polychromatic X-ray in-line phase-contrast imaging. The polychromatic X-ray in-line is based on a light intensity propagation equation proposed by K.A.Nugent in the University of Melbourne Australia. The in-line method is simpler in implementation, as long as the focal point of the X-ray source is small enough, phase-contrast imaging can be achieved on a device based on absorption contrast, however, as a phase second derivative is obtained with the in-line method, it is relatively difficult in phase recovery.

For example, in a Chinese invention patent application with Application Number of 200410053014.6, an X-ray contrast imaging method and system are disclosed. According to the scheme, the sample is imaged on a detector through in-line outline imaging, a distance between a light source point generated by a microfocus X-ray source and a sample on a scanning stage is adjusted according to the sample, and a distance between the sample and the detector is adjusted at the same time. However, an image obtained according to the scheme is a phase second-order differential image of the sample, how phase recovery is carried out to get a phase map of the sample on the basis of the second-order differential image is not described, nor how slice reconstruction and 3D imaging of phase contrast are achieved is described. In addition, as the brightness of the microfocus X-ray source is very low, the detector takes a longer time to expose, and it is difficult to meet the actual needs of clinical applications.

For another example, in a Chinese invention patent application with Application Number of 200810166472.9, an X-ray grating phase-contrast imaging system and method are disclosed. According to the method, phase-contrast imaging under incoherent conditions of approximate decimeter order-of-magnitude field can be achieved by using an X-light machine, a multi-seam collimator such as a source grating, and two absorption gratings. However, in the technical solution, the making of the grating is still a bottleneck, which will restrict actual application of the grating phase-contrast imaging technology in medicine and industry. US 2004/066904A1 discloses a radiation imaging system comprises a movable radiation source adapted to be disposed in a plurality of respective radiation source positions; a radiation detector and a collimator assembly configured to displace a collimator in a plurality of respective collimator positions, each of the collimator positions being coordinated with at least one of the radiation source positions such that a radiation beam emanating from the radiation source is collimated to limit radiation incident on the detector to a predetermined exposure area.

In addition, the existing phase-contrast imaging systems mostly employ a detector based on energy integral, such that the radiation imaging system utilizes the X-rays penetrating the sample at a lower rate. On the other hand, although structural information inside the sample can be obtained by using the detector based on energy integral, the capability of acquiring matter composition information of the sample is insufficient.

In addition, a synchronous radiation X-ray source belongs to a large scientific device, the equipment and maintenance costs are expensive, using it as medical clinical diagnostic equipment is not in line with the principle of effective utilization of energy and resources, and the imaging diagnosis cost cannot be afforded by general patients.

## SUMMARY

With respect to the shortcomings of the prior art, the technical problem to be solved in the present invention is to provide a photon count-based radiation imaging system.

A further technical problem to be solved in the present invention is to provide a photon count-based radiation imaging method.

To achieve the foregoing invention objectives, the present invention adopts the following technical solutions: A photon count-based radiation imaging system, comprising:

an X-ray source used for generating X-rays,

an X-ray collimator used for restricting and adjusting widths and directions of X-ray beams,

a photon count detector used for collecting ray signals produced when the X-rays penetrate an object,

a deflection mechanism and an electron-beam reduction target, and a timing position controller used for used for synchronously controlling irradiation directions of the X-rays according to predetermined timing and activating corresponding partitions of the photon count detector;

wherein the X-ray source generates X-rays, which points to the activated partitions of the photon count detector after adjustment by the X-ray collimator;

the timing position controller activates one partition of the photon count detector; the deflection mechanism adjusts a flight direction of an electron beam, and the electron-beam reduction target causes the electron beam to slow down suddenly and generate X-rays; and with the constraint of the X-ray collimator, the X-rays point to the activated partition of the photon count detector.

Preferably, the X-ray collimator is a micropore collimator, disposed behind the electron-beam reduction target.

A photon count-based radiation imaging method, including:

(1) partitioning the photon count detector;

(2) the timing position controller activating one partition of the photon count detector, and shielding other partitions at the same time;

(3) the X-ray source generating X-rays, which point to the activated partition of the photon count detector after being adjusted by the X-ray collimator;

(4) the photon count detector collecting and recording data of the partitions; and

(5) switching to another partition of the photon count detector, repeating steps (2)-(4), until data collection of all the partitions of the photon count detector has been completed, and obtaining suppressed images of scattered rays.

Preferably, the smaller the partition of the photon count detector is, the lower the rate of contribution of the scattered rays.

Compared with the prior art, the present invention has the following beneficial effects:

(1) the power of the X-ray source can be reduced, and the radiation on the patient is reduced;

(2) in the case of the lowest radiation, structural information inside a diseased area is collected, which may not lead to missed diagnosis;

(3) the data is collected at a faster speed, which is conductive to three-dimensional reconstruction; and

(4) the system is miniaturized and is easy to maintain, which reduces the cost.

## BRIEF DESCRIPTION OF THE DRAWINGS

FIG. 1 is a schematic diagram of the appearance of a photon count detector according to the present invention;

FIG. 2 is a schematic diagram of the structure and the size of the photon count detector according to the present invention;

FIG. 3 is a functional block diagram of a pixel unit of the photon count detector according to the present invention;

FIG. 4 is a schematic circuit diagram of the pixel unit of the photon count detector according to the present invention;

FIG. 5 is a schematic diagram of encapsulation and splicing of a photon count detector module in the prior art;

FIG. 6 is a schematic diagram of a multi-stack encapsulated photon count detector according to the present invention;

FIG. 7 is a schematic structural diagram of large-area plane seamless splicing of the photon count detector according to the present invention;

FIG. 8 is a schematic diagram of pins of the pixel unit of the photon count detector according to the present invention;

FIG. 9 is an overall schematic structural diagram of a photon count-based radiation imaging system, wherein the top right corner is another point of view of some components;

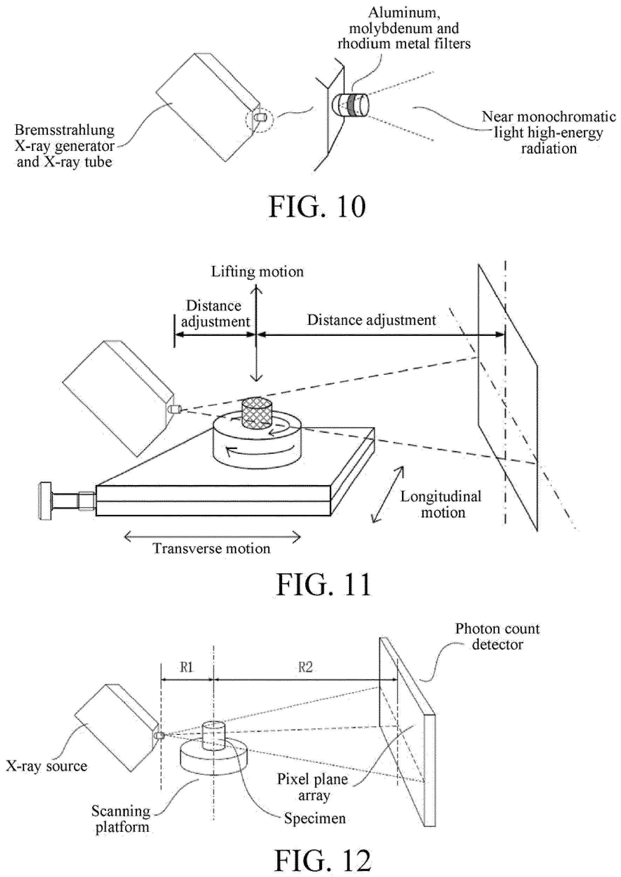

FIG. 10 is a schematic diagram of obtaining annear monochromatic X-ray after filtering of a filter;

FIG. 11 is a structural example diagram of a scanning platform;

FIG. 12 is a schematic diagram of a geometry relation of cone-beam imaging, wherein the relationship among R1, R2, M and the size of the detector is shown;

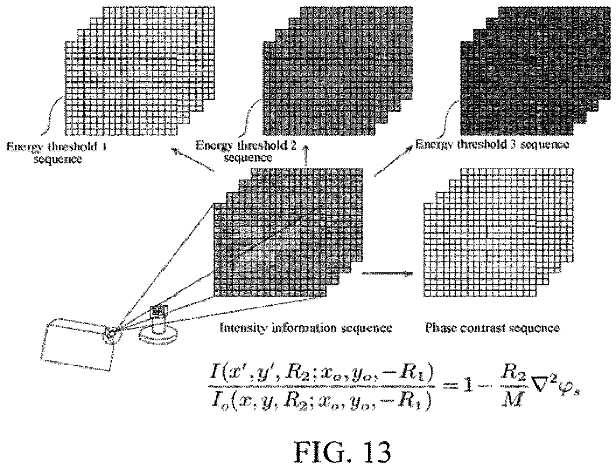

FIG. 13 is a schematic diagram of the working principle of phase-contrast imaging;

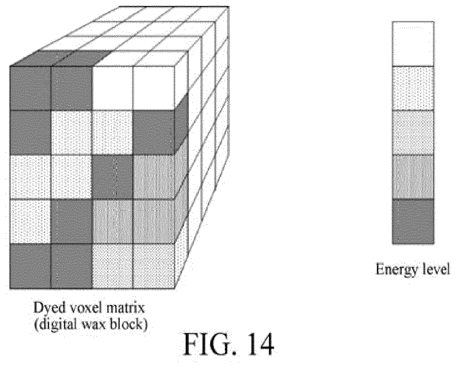

FIG. 14 is a schematic diagram of a digital wax block after digital dyeing;

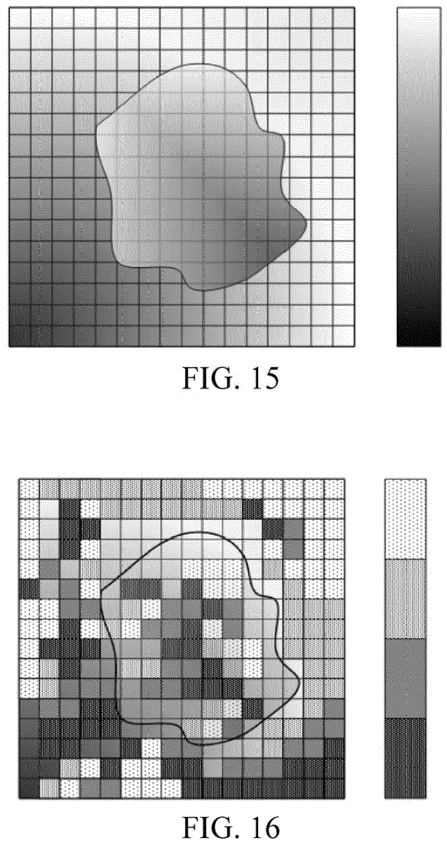

FIG. 15 is a schematic sectional diagram of digital dyeing using intensity information or phase information;

FIG. 16 is a schematic sectional diagram of digital dyeing to which energy level discrimination is added;

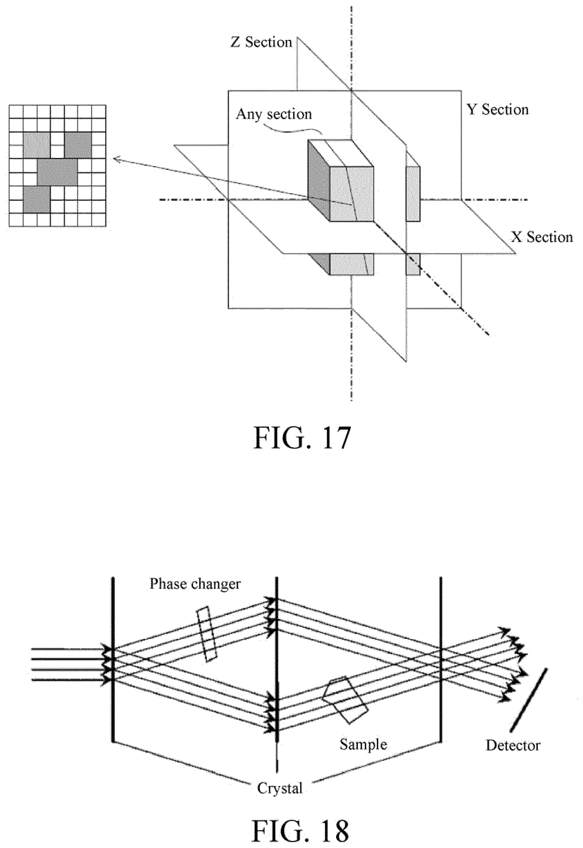

FIG. 17 is a schematic sectional diagram of obtaining any angle from a reconstructed digital was block;

FIG. 18 is a schematic diagram of a basic light path of crystal interference contrast imaging;

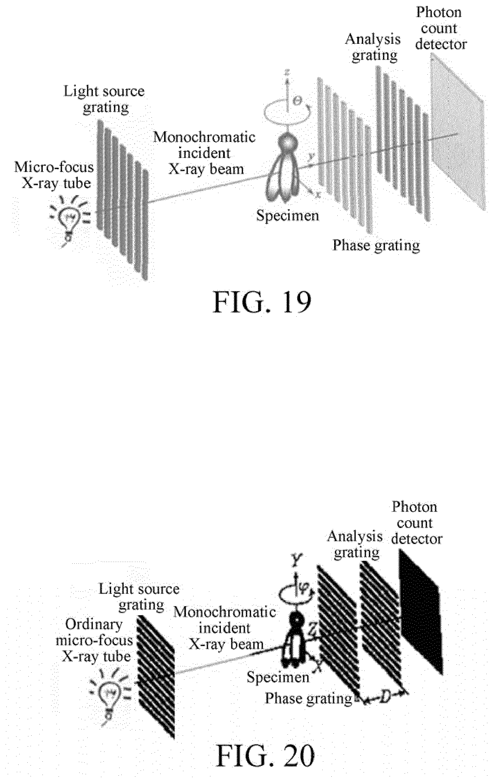

FIG. 19 is a schematic diagram of a grating shearing imaging device that extracts a horizontal refraction angle;

FIG. 20 is a schematic diagram of a grating shearing imaging device that extracts a vertical refraction angle;

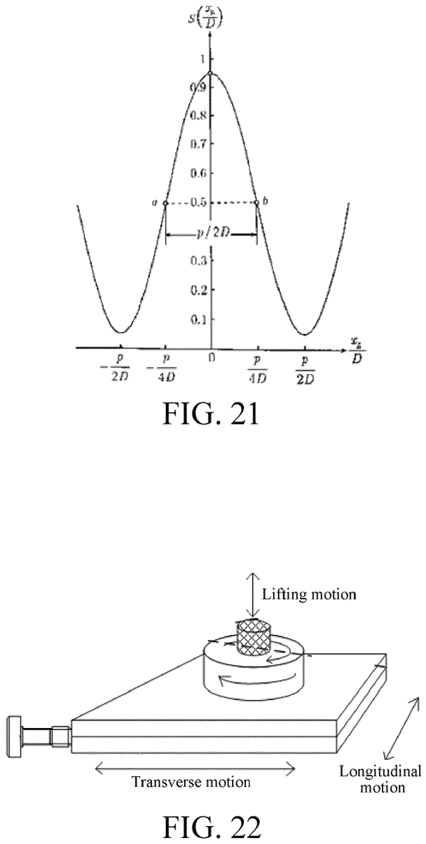

FIG. 21 is a schematic diagram of a displacement curve of an analysis grating in Embodiment 2 of the present invention;

FIG. 22 is a structural example diagram of a sample scanning platform,

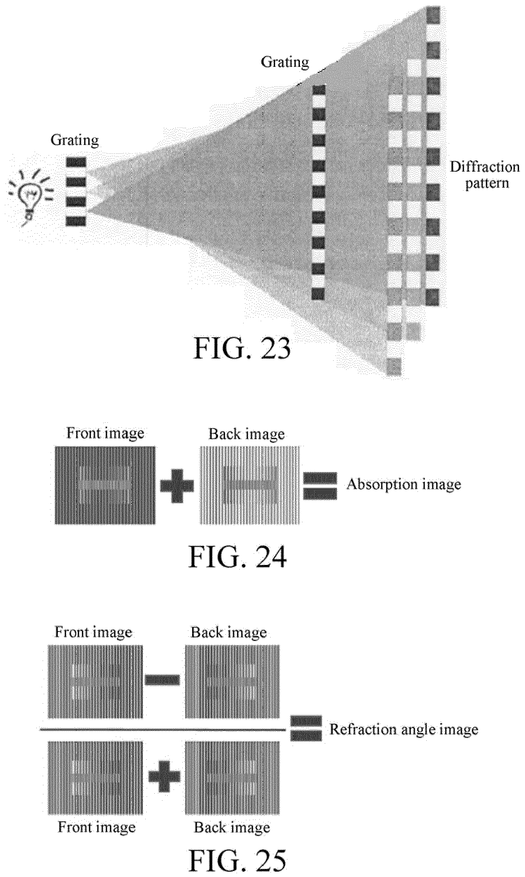

FIG. 23 is a schematic diagram of increase of grating imaging contrast after a light source moves;
FIG. 24 is a schematic diagram of separation and extraction of an absorption image;
FIG. 25 is a schematic diagram of separation and extraction of a refraction image;
FIG. 26 is a schematic diagram of straight ray and scattered ray imaging;
FIG. 27 is a schematic diagram of distribution of scattered rays;
FIG. 28 is an effect diagram of influences of scattered rays when different pixel units are closed;
FIG. 29 is a schematic diagram of a radiation imaging system that employs mechanical control in the Embodiment of the present invention;
FIG. 30 is a schematic diagram of an image collection process in the Embodiment of the present invention;
FIG. 31 is a schematic diagram of a radiation imaging system that employs electronic control in the Embodiment of the present invention;
FIG. 32 is a schematic structural diagram of an electronically controlled X-ray source in the Embodiment of the present invention;
FIG. 33 is a schematic effect diagram of images collected by the radiation imaging system in the prior art; and
FIG. 34 is a schematic effect diagram of images collected by the radiation imaging system in the Embodiment of the present invention.

## DETAILED DESCRIPTION

The technical contents of the present invention are further described below in detail with reference to the accompanying drawings and specific embodiments.

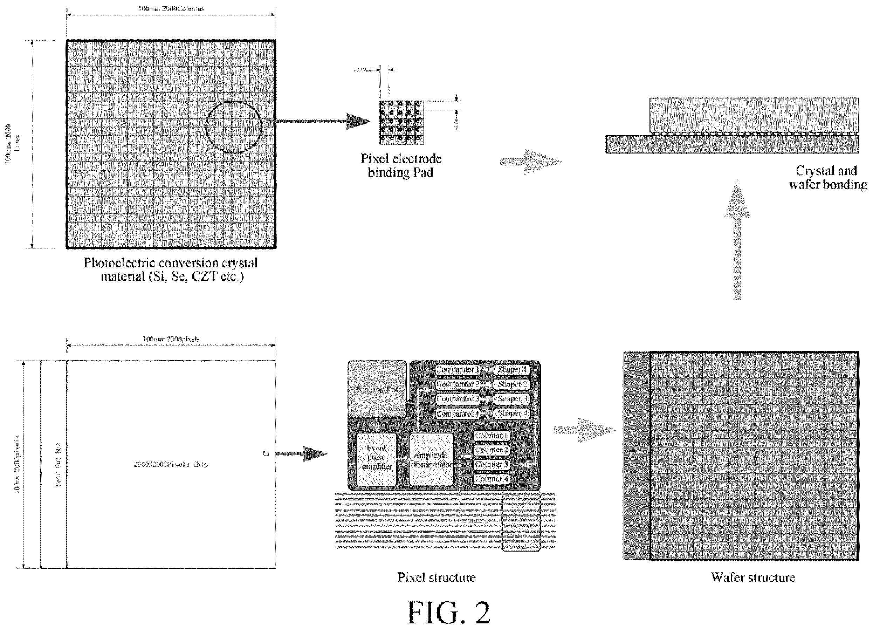

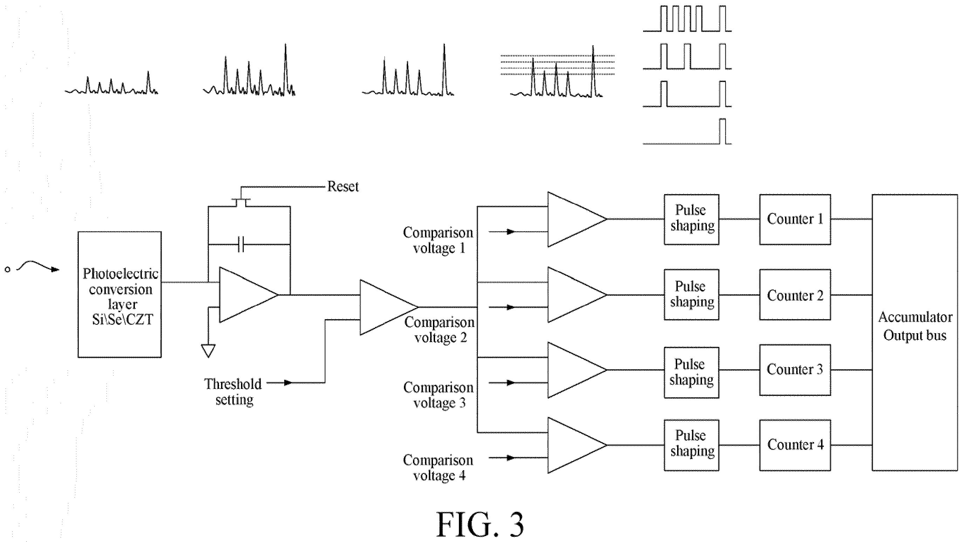

FIG. 1 to FIG. 4 illustrate a new photon count detector according to the present invention. As shown in FIG. 1 and FIG. 2, the photon count detector is a plane-array structure made up of multiple pixel units. FIG. 3 illustrates a functional circuit diagram of each pixel unit of the photon count detector. Each pixel unit includes a photoelectric conversion layer, a pre-amplifier, an event detection unit, a level discrimination comparator, a pulse shaper, a counter, an accumulator and an output bus; wherein the photoelectric conversion layer converts a single photon to an electrical signal, and the electrical signal is transmitted to the pre-amplifier for amplification; the event detection unit filters noise in the electrical signal and sends the electrical signal to the level discrimination comparator; the level discrimination comparator grades effective signals which enter the pulse shaper for pulse shaping; and the counter counts pulse signals which are input to the accumulator and output to the output bus.

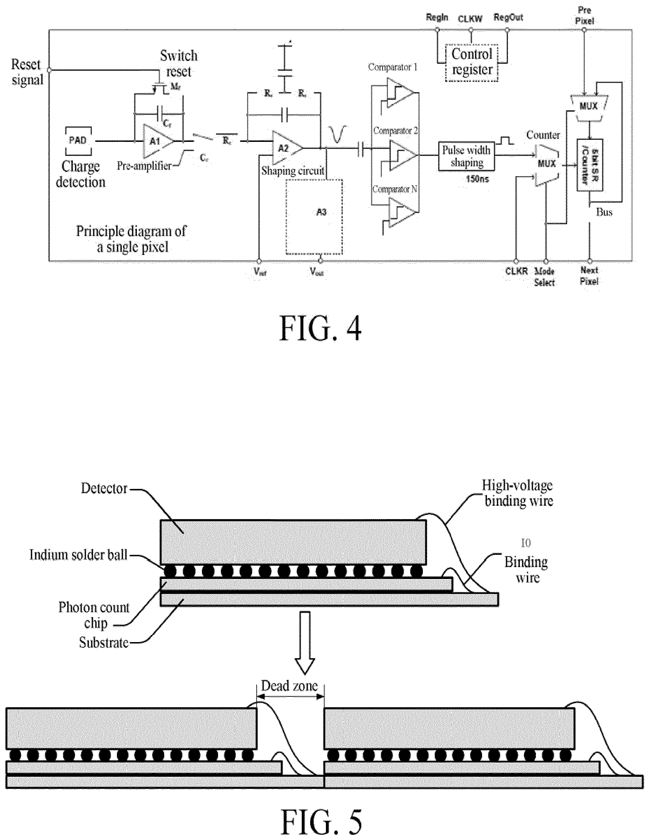

As shown in FIG. 4, the photoelectric conversion layer is connected to an input terminal of the pre-amplifier, an output terminal of the pre-amplifier is connected to the event detection unit, and an output terminal of the event detection unit is connected to the level discrimination comparator. In one embodiment of the present invention, the pre-amplifier, the event detection unit and the level discrimination comparator are all achieved by an operational amplifier and peripheral circuits thereof. The level discrimination comparator is achieved by four comparator circuits connected in parallel. The comparator circuits are respectively provided with a comparison voltage 1, a comparison voltage 2, a comparison voltage 3 and a comparison voltage 4 used as comparison references, and output terminals of the comparator circuits are connected with the pulse shaper and the counter sequentially. Output terminals of the four counters are connected with the accumulator and the output bus respectively. In addition, the detector unit may also be provided with several (generally 5) registers, wherein the total number of photon events is stored in one of the registers, and the number of photons at different energy levels is stored in others.

In the photon count detector according to the present invention, the photoelectric conversion layer is used for achieving conversion of optoelectronic signals, and the material used may be one of silicon, cadmium telluride, cadmium zinc telluride and selenium, which is not specifically defined in the present invention. In the photoelectric conversion layer, single X-photons are captured, and an electron hole pair is formed. The electron hole pair, under the action of an applied electric field, is transmitted to the input terminal of the pre-amplifier. The pre-amplifier pulse-amplifies a single photon event, which is handed over to the event detection unit for filtering noise. After the X-rays penetrate the sample, as atomic numbers of tested materials on the light path are different, energy of photons reaching the surface of the photon count detector varies, and event pulse amplitudes formed are also different. Event pulses and a set threshold are discriminated and compared, and low-energy pulses can be discriminated and filtered. The function of the level discrimination comparator is distinguishing an effective event pulse from random noise and threshold-comparing the effective event pulse. Suppose that the number of the level discrimination comparator is set as K (K is a positive integer), the energy of a single X-photon may be divided into K+1 level discrimination groups. After the effect of the pulse shaper on the output of each level discrimination comparator, the event pulse is shaped into one channel pulse output, in order to perform subsequent signal digital processing. After the effect of the pulse shaper on each level event pulse, the counter counts photon events at different levels. Within a set counting time cycle, photon events of each channel are accumulated, and the cumulative sum is concurrently transmitted to an external data processing device within a readout cycle of the output bus. The counting cycle differs between $1/10^8$ s and 1 s, which is decided according to sizes of photon streams and significant digits of the counter and is also set according to actual application. Bus readout of each pixel unit merely requires 1 clock cycle for concurrent transmission, all the counters are reset simultaneously with the readout, and next, photon events are counted continuously. The timing can be controlled, so as to be used in different application occasions. Duty ratios of the counting and the readout may also be adjusted. Data of each pixel unit includes the counting sum and the cumulative sum of multiple energy levels. The counting sum indicates photon event energy information on the pixel unit, and the cumulative sum indicates density-related absorption attenuation information obtained by the pixel unit.

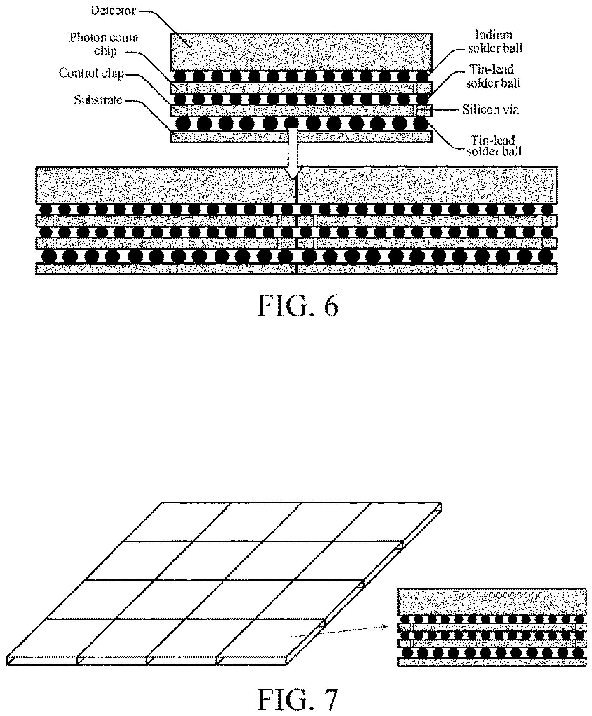

As shown in FIG. 5, when the photon count detector module performs array splicing, as enough space should be reserved for a routing area, a problem of splicing dead-zone definitely exists, and with respect to the above technical problem, the present invention provides a photon count detector. As shown in FIG. 6, the photon count detector provided in the present invention can achieve large-area plane seamless splicing, including a detector module, a photon count module, a control module and a substrate, and multi-stack encapsulation is achieved through methods such as a silicon via, indium solder ball flip-chip, tin-lead solder ball flip-chip and the like. The top layer is the detector module, which is connected with the photon count module on the lower layer through an indium solder ball. The photon count module in the present invention not only includes a pixel unit array, achieving functions of low-noise amplification of detector signals, threshold comparison, counting and data readout. The size of the photon count module is exactly the same as that of the detector module. Through the silicon via, a data input/output interface, a power supply interface, a bias interface, a control interface and the like of the photon count module are led out to the back of the photon count module, which is connected with the control module on the lower layer through an indium solder ball. The control module supplies power for the photon count module, provides a bias voltage, and completes a reading/writing function and an input/output buffer function. The control module also leads the input/output interface to the back of the chip through a silicon via technology, and then is connected to the substrate through tin-lead solder ball flip-chip. By use of such a multi-stack encapsulation form, the problem of splicing dead-zone of the traditional photon count detector can be solved, to achieve seamless splicing between the modules.

As shown in FIG. 7, considering that the detector module still needs to lead a high-voltage offset line out, if large-area seamless splicing is to be achieved, the detector module array can still only use the form of 2*N (N is a positive integer) array. The photon count module and the control module can use the form of M*K (M and K are positive integers) array. M=2*L, K=N*S (L and S are positive integers), that is, one detector module is flipped with L*S photon count modules. In one embodiment of the present invention, it is feasible to set N=2, L=2 and S=2, that is, each detector module and four photon count modules achieve encapsulation in the form of 2x2 array to form a photon count detector module, the size of each photon count module is 15 mm* 15 mm, the size of each detector module is 30 mm*30 mm, and the size of each photon count detector module is 30 mm*30 mm. The photon count detector module is spliced according to the form of 2x2 array, and the size of the entire detector plane-array is 60 mm*60 mm. Such a large detector array plane can meet some imaging application demands, if it is necessary to increase the detector array plane, the detector module array should be ensured as in the form of 2*N, the size of the detector module can be increased to 60 mm*30 mm, each detector module is flipped with 4*2 photon count modules, to form a detector module with the size of 60 mm*30 mm, the detector module is used to perform splicing in the form of 2*4 array, and the entire detector array plane can be up to 120 mm* 120 mm.

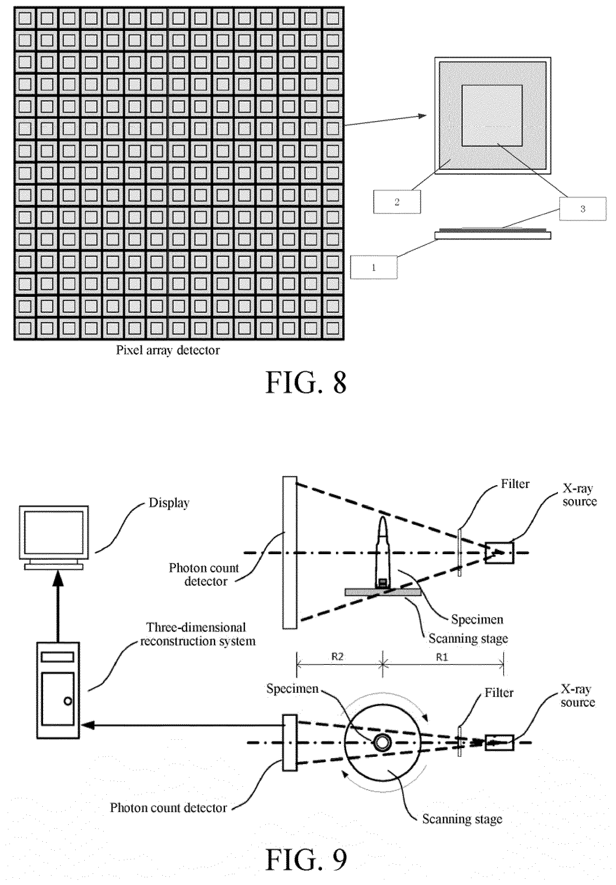

FIG. 8 illustrates a detector module structure. Each detector module is made up of a matrix type diode array. FIG. 8 gives a planar structure of a single PN-junction diode detector. In FIG. 8, the area denoted by the reference sign 1 represents an N-type substrate, the area denoted by the reference sign 2 represents a P+ doped area, and the area denoted by the reference sign 3 represents apositive-electrode pad. The N-type substrate is a common cathode of all diode arrays, and in an actual application, the common cathode is connected to a positive high-voltage end. In one embodiment of the present invention, the detector module is made of a high-resistance n-type silicon wafer, and according to an energy range of the X-rays to be detected, the thickness of the wafer may be selected as 300 $\mu$m, 500 $\mu$m, 700 $\mu$m, 1 mm or the like. The spacing of each pixel unit is 330 $\mu$m, the area of the P+ doped area is 300 $\mu$m *300 $\mu$m, and the area of the positive-electrode pad is 180 $\mu$m *180 $\mu$m. The entire detector module is in the form of 90*90 array, the area of the core pixel unit area is 29.7 mm*29.7 mm, the boundary of the pixel unit is at a distance of 150 $\mu$m from the edge of the detector module, and the size of the complete detector module is 30 mm*30 mm.

In one embodiment of the present invention, the photon count module array is 45*45, the size of the single pixel unit is 330 $\mu$m *330 $\mu$m, and the size of the entire chip is 15 mm*15 mm.

On the basis of the above photon count detector, the radiation imaging system and the radiation imaging method can be implemented in many manners.

Embodiment of the Invention

As the grid of the X-ray source in the prior art has the following shortcomings: on the one hand, as the focal point of the grid is fixed, imaging requirements for different parts cannot be met, and the imaging quality is seriously affected; on the other hand, the grid per se also blocks some X-rays that should be shot to the photon count detector. In order to eliminate such adverse effects, radiation dose of the X-ray must be increased, which causes more radiation to tested objects especially patients and medical staff, increases the manufacturing cost of the photon count detector, and brings about difficulty to environmental protection work of the hospital.

As the virtual grid technology in the prior art does not filter scattered rays reaching the photosensitive plane of the photon count detector, scattered ray and straight ray data are all sampled. For the thick position radiography where scattered rays take a greater proportion, minor details of the straight rays have been "flooded" by lots of scattered rays when reaching the photon count detector, the minor details cannot be restored through software processing, and medical diagnosis requirements of thick position applications cannot be met.

There are some scatter correction methods in the prior art, which generates a scattering intensity distribution map by using a scatter corrector or an attenuation grid, and obtains a corrected projection image through a difference between a projection image and the scattering intensity distribution map. However, the scatter correction methods may double the scanning time, also double the data processing volume is also doubled, and have problems such as low efficiency and poor adaptability.

Therefore, to solve the above technical problems, the present invention further provides aphoton count-based radiation imaging system and method.

According to some known research results (specifically refer to a Chinese invention patent with Patent Number of ZL 200910022100.3), distribution of scattered rays may be approximately regarded as following normal distribution. For the one-dimensional situation, after one narrow-beam X-ray penetrates the tested object, intensity distribution of rays (including straight rays and scattered rays) on various positions of the x direction may be shown as the formula (10):

$$f(x) = \frac{1}{\sqrt{2\pi}\sigma} \exp\left(-\frac{(x-\mu)^2}{2\sigma^2}\right) \qquad (10)$$

σ denotes the characteristic of the tested object, the size is determined by density and thickness thereof, and u denotes the position of the narrow-beam ray in the x direction.

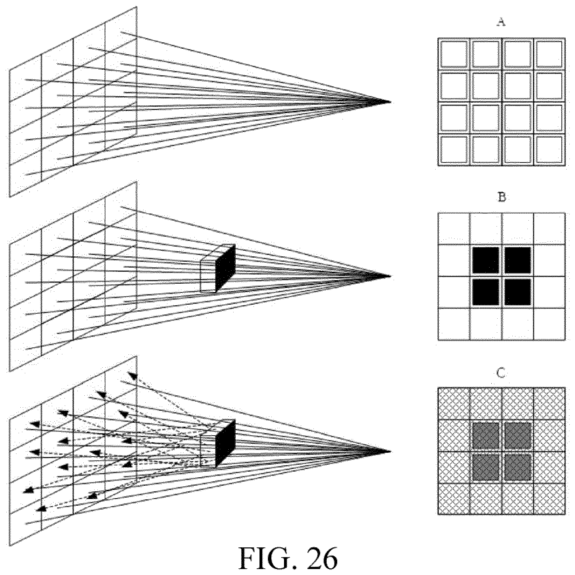

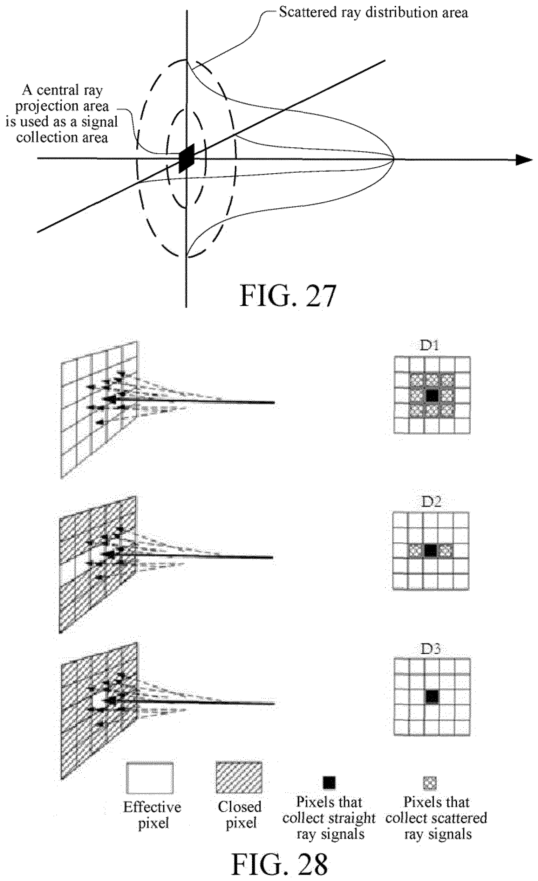

As shown in FIG. 26 and FIG. 27, when straight rays from the focal point are transmitted to the photosensitive plane (preferably a scintillant coating and a thin film transistor array) of the photon count detector, scattered rays may be formed during the travel, thus forming the scattered ray distribution map shown in FIG. 26 and FIG. 27. The black block in the central position is a central ray projection area, and it is used as a signal collection area to collect straight rays from the focal point. The area represented by the circle around the central position is a scattered ray distribution area, and the scattered rays are mainly distributed in the area. In the present invention, to ensure the quality of collected images, it is necessary to perform certain processing on the scattered rays. Specific description is as follows:

The present invention uses an X-ray source performing scanning according to certain timing and a photon count detector that can control the collection position, by controlling the operation timing of the X-ray source and the photon count detector, ensures that only a small enough area obtains exposure of the X-ray in a moment, and only the pixel unit of the area of the photon count detector is in a collection state, while other pixel unit areas of the photon count detector are in a non-response state. In this way, the contribution of the scattered rays to an effective collection area will be reduced greatly. Theoretically, when the straight ray merely points to a single pixel unit and only the single pixel unit is in the collection state, the contribution of the scattered rays approaches zero. Still by taking one dimension as an example, the relationship between the contribution rate of the scattered rays and the size of the collection area can be represented with formula (11):

$$P(D) = \left.\int_{m-D/2}^{m+D/2} e^{-\frac{(x-m)^2}{2\sigma^2}} dx \middle/ \int_{-\infty}^{+\infty} e^{-\frac{(x-m)^2}{2\sigma^2}} dx \right. \qquad (11)$$

*m* denotes the position of the narrow-beam ray in the x direction; D denotes the collection width, taking *m* as the center; and P denotes the contribution rate of the scattered rays.

It can be known through the formula (11) that the smaller the collection width is, the smaller the contribution rate of the scattered rays is. In the case that only a single pixel unit or a small area collects directional X-rays at each moment while other areas do not collect X-rays, and scattering of X-rays in other directions affects scattering components of effective collection pixel units to be almost zero, signals collected by pixel units in an effective collection area (that is, effective collection pixel units) or a single effective pixel unit will be completely from straight rays.

As shown in FIG. 28, single-beam X-rays are shot to different areas (pixel units or pixel unit blocks) on the photon count detector. The white square on the photon count detector represents an operating pixel unit, and the shaded square represents a pixel unit that has been closed but does not operate. In collected images D1-D3, the black square represents a pixel unit that collects straight ray signals, and the shaded square represents a pixel unit that collects scattered ray signals. The situation shown by the image D1 is the images collected by all the pixel units when in the operating state. When an X-ray is shot to the photon count detector, some rays may change directions to form scatted rays. When the X-ray reaches the photosensitive plane of the photon count detector, the situations of the pixel units affected are as shown by D1 in the figure. In addition that the pixel unitof the photon count detector collects straight rays, pixel units (that is, the shaded pixel units in FIG. 28) around the pixel unit may also collect signals of the scattered rays. The situation shown by the image D2 represents straight ray signals and scattered ray signals collected by the photon count detector when only one row of pixel units are activated and other pixel units are closed. Compared with the image D1, the scattered rays collected by the image D2 are evidently reduced. The situation shown by the image D3 is that the photon count detector does not acquire scattered signals when only one pixel unit is activated. In the present invention, collection is not necessary for the pixel units except the effective collection pixel units (that is, the pixel units shown by the black squares in FIG. 28), that is, the surrounding effective pixel units will not collect or transmit the scattered rays even if receiving the scattered rays. It can be known from theoretical derivation that, when a bundle of rays are limited to only exposing a single pixel unit, other pixel units can only receive scattered rays, while the pixel units that the bundle of rays face only have signals from primary rays, but scattered ray signals do not exist.

The radiation imaging system of this embodiment performs filtering and scattering by using a photon count detector and a time-sharing partition control manner, to increase the imaging effect, which includes an X-ray source, an X-ray collimator, a photon count detector, a timing position controller and other components.

(1) The X-ray collimator is used for restricting and adjusting widths and directions of X-ray beams. After the X-ray source is processed by the X-ray collimator, it is feasible to emit directional X-rays only to pixel unit areas (rows, points or small blocks) in the photon count detector which are in a state of responding to the X-rays, and straight ray parts of the X-rays will reach the pixel units in the photon count detector which are in a response state, while scattered rays will reach pixel unit areas which are in a state of not responding to the X-rays.

(2) The photon count detector can perform scanning and collection row by row, point by point or block by block in a time-sharing manner, in each time period, only one pixel unit or one small area (n*m pixel unit blocks, n and m are positive integers) is in a state of responding to the X-rays, while other pixel units or areas are all in a state of not responding to the X-rays.

(3) The timing position controller can control each area (row, point or small block) of the photon count detector to be in a collection state, control other partitions to be in a non-collection state (masked state), control X-rays emitted by the X-ray source to point to effective collection areas of the photon count detector, and ensure that no rays are emitted in other directions.

This embodiment further provides a photon count-based radiation imaging method, implemented based on the above photon count-based radiation imaging system, including:

(1) partitioning the photosensitive plane of the photon count detector;

(2) the timing position controller activating one partition of the photon count detector, and shielding other partitions at the same time;

(3) controlling the X-ray source to emit X-rays, which point to the activated partition after being restricted by the X-ray collimator;

(4) thephoton count detector collecting and recording data of the partition; and

(5) switching to another partition of the photon count detector, repeating steps (2)-(4), until data collection of all the partitions of the photon count detector has been completed. Thus, a complete scattered image can be obtained.

In this embodiment, the timing position controller partitions the photosensitive plane of the photon count detector according to the control mode selected by the user, and synchronously controls radiation directions of the X-rays according to partitioning results and activates corresponding partitions of the photon count detector. In this embodiment, a preferred scheme is that the timing position controller consists of a dot frequency generation circuit, a point counting circuit, a row counting circuit, a frame frequency control circuit, a detector timing control output circuit, an X-ray position control output circuit and the like. The circuits can be formed by slight transforming the timing controller or the timing control circuit in the existing CRT display. As a conventional technology that can be grasped by those skilled in the art, the circuits are not specifically described herein.

The radiation imaging system provided in this embodiment can employ two control modes: a mechanical control mode and an electronic control mode. Specific technical contents of the two control modes are specifically described below in detail respectively.

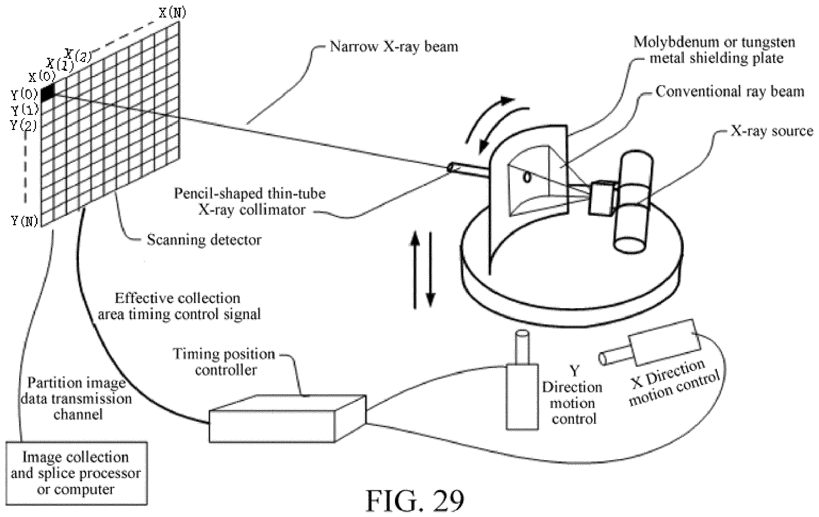

As shown in FIG. 29, when the radiation imaging system of this embodiment employs the mechanical control mode, a corresponding mechanical motion device is further included. The photon count detector may be used in a time-sharing and partitioning manner, that is, the photon count detector uses a pixel unit as the minimum unit, and it is feasible to use a single pixel unit, it is also feasible to use a single row of pixel units or multiple rows of pixel units, and it is also feasible to use a pixel unit block formed by several adjacent pixel units; the mechanical motion device includes drive motors in the X direction and the Y direction (or X direction, Y direction and Z direction), and thus under the guide of a control command, it is feasible to move in the X direction and the Y direction; the X-ray collimator is specifically a pencil-shaped thin-tube X-ray constraint in this embodiment, used to constrict X-rays to make the X-rays form a narrow X-ray beam. It can move under the control of the mechanical motion device; and the timing position controller is used for partition-activating the photon count detector according to certain timing.

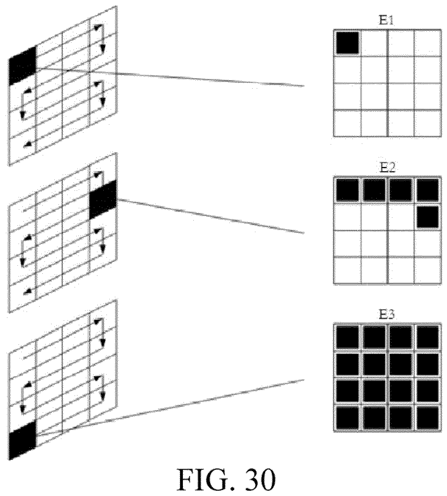

In the case of the mechanical control mode, the working principle of the radiation imaging system is as follows: the X-ray source obtains a small enough narrow X-ray beam through a pencil-shaped thin-tube X-ray constraint, points to an activated area of the photon count detector, and only exposes one particular position within unit time. Within the unit time, the corresponding pixel unit in the photon count detector is notified to perform activation through a position signal, and signals of the area are collected. As shown in FIG. 30, the pencil-shaped thin-tube X-ray constraint, under the control of the mechanical motion device, moves along the X positive direction through the drive motor in the X direction, and synchronously moves with the activated area of the photon count detector, until all the pixel units in the X positive direction are completely exposed. Next, the drive motor in the Y direction is moved to a height of one area along the Y direction, to continuously perform scanning and exposure in the X negative direction. After walking all the way in the Y direction, each area of the photon count detector is completely exposed. The exposure process ensures that, under the directing of each mechanical motion, merely the directed pixel unit is activated, and ensures that only the activated pixel unit can collect and transmit straight X-ray signals, while other pixel units are in a closed or non-activated state and may not collect or transmit scattered rays received around.

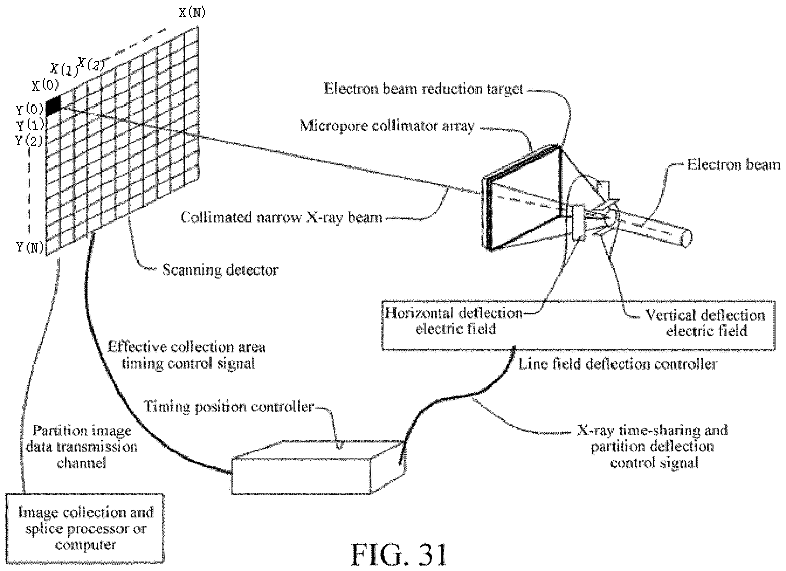

When the radiation imaging system provided in this embodiment employs the electronic control mode, as shown in FIG. 31, an electronic gun, an electron-beam reduction target and a deflection mechanism. The photon count detector can be used in a time-sharing and partitioning manner, that is, the photon count detector uses a pixel unit as the minimum unit, and it is feasible to use a single pixel unit, a single row of pixel units or multiple rows of pixel units, and it is also feasible to use a pixel unit block formed by several adjacent pixel units; the X-ray source is replaced with an electronic gun in this embodiment. The electronic gun is used for emitting electron beams. The electron beams are controlled by an electromagnetic field, and the movement direction thereof can be adjusted. The deflection mechanism is used for adjusting the directions of the electron beams and making them point to corresponding partitions; the electron-beam reduction target (e.g., tungsten target or molybdenum target) is disposed in a vacuum environment, used for making the electron beams suddenly slow down and generating X-ray beams during reduction of the electron beams; the X-ray collimator is specifically a micropore collimator (also referred to as a partition collimator), disposed behind the electron-beam reduction target, used for collimating the X-ray beams; and the timing position controller is used for partition-activating the photon count detector according to certain timing.

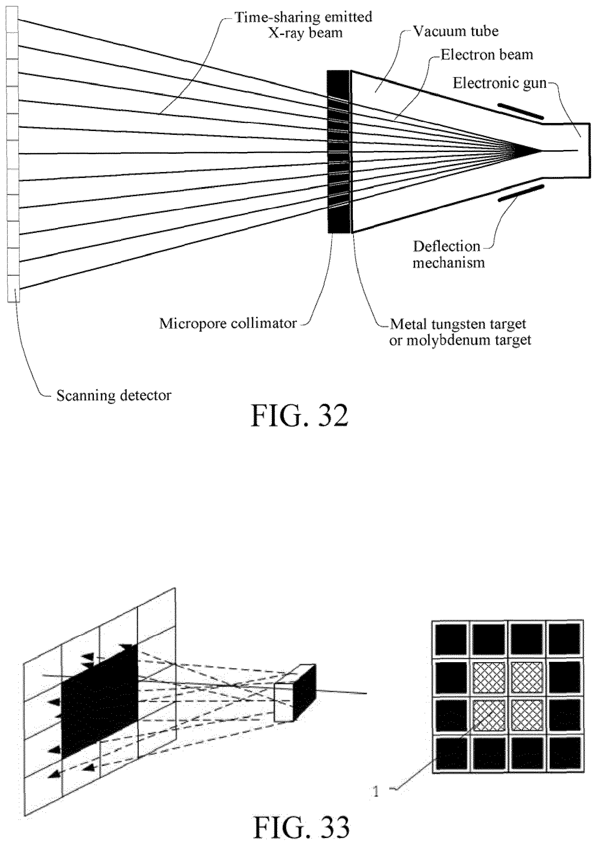

As shown in FIG. 32, when the radiation imaging system employs the electronic control mode, the working principle is as follows: through the command of the timing position controller, electrons emitted by the electronic gun form an electron beam, which deviates from the original movement direction under the action of the deflection mechanism, bombards the tungsten target in the corresponding area and generates X-rays, the X-rays pass through the micropore collimator and points to a first area of the first row on the photon count detector, and the timing controller activates pixel units in the first area of the first row on the photon count detector at the same time, to acquire non-scattered images of the first area of the first row. At this point, pixel units adjacent to the activated area may be subject to radiation of scattered rays, and as the pixel units are not activated, information of the scattered rays are not collected and transmitted. According to the timing command of the timing position controller, the photon count detector will complete collection of remaining areas of the first row one by one. When the pixel units of the first row are completely collected, pixel units of the second row are collected inversely and so on, until pixel units of all the rows are completely collected, thus obtaining a clean image where scattered rays are suppressed. The process is the same as the line of collecting images in the case of mechanical control mode, and is not repeated herein.

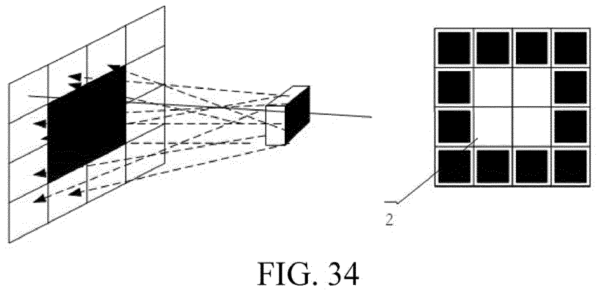

As shown in FIG. 33, the photon count detector in the traditional plane array collection mode, due to its inherent defects, will collect straight rays and scattered rays at the same time, making images collected by the photon count detector polluted, for example, shown by the area represented by reference sign 1 in FIG. 33. Images collected by the radiation imaging system provided in the present invention are as shown by the area represented by reference sign 2 in FIG. 34. It can be seen therefrom that, compared with the photon count detector in the traditional plane array collection mode, image collection achieved by the present invention significantly suppresses the scattered rays, and the image contrast and the signal-to-noise ratio are remarkably increased.

In actual use, it is difficult for the collection width to control the size of one pixel unit, and it is necessary to make a balance between the collection width and the contribution rate of the scattered rays. When a bettered de-scattering effect is required, in the case that the requirement for the collection speed is not high, it is feasible to choose a narrower X-ray beam as much as possible and simultaneously activate a smaller pixel unit area, and at this point, the pixel unit area may be one row of pixel units, multiple rows of pixel units, a pixel unit block formed by several adjacent pixel units, and at least, may even be one pixel unit; and vice versa. The order in which the pixel units are activated may be in a manner of first along the X direction and then along the Y direction, and may also be in other preset manners.

When images are collected point by point by taking a single pixel unit as an irradiation area, at this point, only one area (minimize to one pixel unit) is allowed to be in an activated state at each time, therefore, suppression capability for surrounding scattered rays is the strongest, the quality of the images collected is very high, but the collection speed is slow, which can be applied to occasions where the requirement for the collection speed is not very high. When line-by-line scanning is performed, as the line-by-line scanning mode is a state in which all pixel units in the line are activated simultaneously at the same time, there are still a small number of scattered rays collected by adjacent pixel units in the same line during line exposure and data collection. As the line-by-line scanning mode has a faster collection speed, in some situations where fast collection is required but the requirement for the image quality is not high, the line-by-line scanning mode is meaningful. In this way, the present invention can the demand for the image collection speed in different situations, thus obtaining images in line with actual demands, and even collect de-scattered images with higher precision.

For collection of multiple pixel units, it is feasible to increase one pre-processing module to solve the situation where the images are polluted. After each detector module preliminarily integrates the collected original data, the data is input to multiple photon count detector collection circuits, and then is sent to the pre-processing module via multiple data transmission channels. The pre-processing module may process, such as integrate, rearrange and correct, data frames, and the pre-processing module may also de-scatter and filter the data frames according to a de-scattering algorithm, to discriminate and discard related scattered data frames, but only retain effective data frames. The de-scattering algorithm of the pre-processing module may be implemented through FPGA, DSP and even an ASIC chip. The discriminated effective data frames are then sent to the host machine for concurrent image reconstruction. The de-scattering function of the scheme is implemented in the pre-processing module, there is no special requirement for the photon count detector, it is feasible to use the traditional photon count detector module, and it is unnecessary to redesign the photon count module.

In addition, according to the scheme, it is unnecessary to reconfigure the register during each scanning, and the scanning speed can be ensured, but the frame rate is not reduced. It is more flexible to implement the de-scattering function through FPGA, DSP and other programmable logic devices, different application demands and scanning modes can be implemented through hardware programming, to enable a set of detector systems to be compatible with multiple operation modes, greatly improving application flexibility of the detector systems. At the same time, the reconstructed original data received by the host machine is pre-processed, which reduces the data volume, simplifies complexity of the image reconstruction algorithm, and can reduce the requirement of the host machine for image reconstruction hardware resources.

This embodiment can adapt to detection requirements of different parts without increasing the radiation dose. At the same time, in the case of the same scanning time, the data processing volume is greatly reduced, the image effect is evidently increased, tiny details can be restored, and the requirement of medical diagnosis is fitted. During X-ray imaging in the case of reconstruction of two-dimensional images and three-dimensional images, by use of the method provided in the present invention, influences of scattered rays on images can be reduced, the image signal quality is enhanced, and the radiation dose can be reduced indirectly.

For a designed photon count detector hardware system, to keep integrity of the system, when the system is upgraded, it is feasible to adopt a scheme of software de-scattering.

The original data of the photon count detector unit goes through data pre-processing and frame data pre-processing sequentially, and then is sent to the host machine through a high-speed optical fiber transmission channel. After the host machine receives the frame data, a scattering and screening program is executed through a software algorithm, and the frame data, after scattering and screening, is image-reconstructed. The software algorithm is similar to the hardware algorithm of the pre-processing scheme, but is only achieved through different programming languages.

The biggest advantage to the scheme is that it is unnecessary to make any change to the hardware system and it is feasible to be compatible with the existing photon count detector system, which greatly reduces the hardware cost of the system. At the same time, the software de-scattering scheme has a shorter development cycle, and can get the product to the market faster. However, due to the absence of hardware-level pre-processing, data transmission pressure is increased. Meanwhile, higher requirements are proposed for hardware resources of the host machine.

**Claims**

1. A photon count-based radiation imaging system, comprising:

   an X-ray source used for generating X-rays,
   an X-ray collimator used for restricting and adjusting widths and directions of X-ray beams,
   a photon count detector used for collecting ray signals produced when the X-rays penetrate an object,
   a deflection mechanism and an electron-beam reduction target, and
   a timing position controller configured for synchronously controlling irradiation directions of the X-rays according to predetermined timing and activating corresponding partitions of the photon count detector;
   wherein the X-ray source is configured to generate X-rays, which point to the activated partitions of the photon count detector after adjustment by the X-ray collimator; **characterized in that**
   the timing position controller is configured to activate one partition of the photon count detector; the deflection mechanism is configured to adjust a flight direction of an electron beam towards the electron-beam reduction target, the electron-beam reduction target causing the electron beam to slow down suddenly and generate X-rays; such that with the constraint of the X-ray collimator, the X-rays point to the activated partition of the photon count detector.

2. The radiation imaging system according to claim 1, wherein
   the X-ray collimator is a micropore collimator, disposed behind the electron-beam reduction target.

3. A photon count-based radiation imaging method, using the radiation imaging system according to claim 1 or 2, comprising the steps of:

   (1) partitioning the photon count detector;
   (2) the timing position controller activating one partition of the photon count detector;
   (3) the X-ray source generating X-rays, which point to the activated partition of the photon count detector after being adjusted by the X-ray collimator;
   (4) the photon count detector collecting and recording data of the partitions; and
   (5) switching to another partition of the photon count detector, repeating steps (2)-(4), until data collection of all the partitions of the photon count detector has been completed, and obtaining suppressed images of scattered rays.

4. The radiation imaging method according to claim 3, wherein
   the smaller the partition of the photon count detector is, the lower the rate of contribution of the scattered rays.

**Patentansprüche**

1. Auf Photonenzählung basierendes Strahlungsabbildungssystem, umfassend:

   eine Röntgenquelle, die zur Erzeugung von Röntgenstrahlen verwendet wird,
   ein Röntgenkollimator, der zur Begrenzung und Einstellung der Breiten und Richtungen von Röntgenstrahlen verwendet wird,
   einen Photonenzähldetektor, der zur Erfassung von Strahlensignalen verwendet wird, die beim Durchgang von Röntgenstrahlen durch ein Objekt entstehen,
   einen Ablenkungsmechanismus und ein Elektronenstrahl-Reduktionstarget, und
   eine Zeitpositionssteuerung, die so ausgelegt ist, dass sie die Bestrahlungsrichtungen der Röntgenstrahlen gemäß einer vorgegebenen Zeitsteuerung synchron steuert und die entsprechenden Partitionen des Photonenzähldetektors aktiviert;
   wobei die Röntgenquelle so ausgelegt ist, dass sie Röntgenstrahlen erzeugt, die nach Einstellung durch den Röntgenkollimator auf die aktivierten Partitionen des Photonenzähldetektors ausgerichtet sind; **dadurch ge-**

**kennzeichnet, dass**

die Zeitpositionssteuerung so konfiguriert ist, dass sie eine Partition des Photonenzähldetektors aktiviert; der Ablenkmechanismus so konfiguriert ist, dass er die Richtung eines Elektronenstrahls auf das Elektronenstrahl-Reduktionsziel einstellt,

das Elektronenstrahl-Reduktionsziel, das den Elektronenstrahl abrupt abbremst und Röntgenstrahlen erzeugt, sodass die Röntgenstrahlen unter dem Einwirken des Röntgenkollimators auf die aktivierte Partition des Photonenzähldetektors treffen.

2. Strahlungsabbildungssystem nach Anspruch 1, wobei der Röntgen-Kollimator ein Mikropore-Kollimator ist, der hinter dem Elektronenstrahl-Reduktionsziel angeordnet ist.

3. Auf Photonenzählung basierendes Strahlungsbildgebungsverfahren unter Verwendung des Strahlungsbildgebungssystems nach Anspruch 1 oder 2, das die folgenden Schritte umfasst:

(1) Partitionierung des Photonenzähldetektors;

(2) Steuerung der Zeitposition, die eine Partition des Photonenzähldetektors aktiviert;

(3) die Röntgenquelle, die Röntgenstrahlen erzeugt, die auf die aktivierte Partition des Photonenzähldetektors gerichtet sind, nachdem sie durch den Röntgenkollimator ausgerichtet wurden;

(4) der Photonenzähldetektor, der die Daten der Partitionen sammelt und aufzeichnet; und

(5) Wechsel zu einer anderen Partition des Photonenzähldetektors, Wiederholung der Schritte (2)-(4), bis die Datenerfassung aller Partitionen des Photonenzähldetektors abgeschlossen ist, und Gewinnung unterdrückter Bilder von Streustrahlen.

4. Strahlungsbildgebungsverfahren nach Anspruch 3, wobei gilt: Je kleiner die Partition des Photonenzählers ist, desto geringer ist der Beitrag der gestreuten Strahlen.

**Revendications**

1. Système d'imagerie par rayonnement basé sur le comptage de photons, comprenant :

une source de rayons X utilisée pour générer des rayons X,

un collimateur de rayons X utilisé pour limiter et ajuster les largeurs et les directions des faisceaux de rayons X,

un détecteur de comptage de photons utilisé pour collecter les signaux de rayons produits lorsque les rayons X pénètrent un objet,

un mécanisme de déviation et une cible de réduction du faisceau d'électrons, et

un contrôleur de position temporelle configuré pour commander de manière synchronisée les directions d'irradiation des rayons X en fonction d'une synchronisation prédéterminée et pour activer les partitions correspondantes du détecteur de comptage de photons ;

dans lequel la source de rayons X est configurée pour générer des rayons X qui pointent vers les partitions activées du détecteur de comptage de photons après ajustement par le collimateur de rayons X ; **caractérisé en ce que**

le contrôleur de position temporelle est configuré pour activer une partition du détecteur de comptage de photons ; le mécanisme de déviation est configuré pour ajuster une direction de vol d'un faisceau d'électrons vers la cible de réduction du faisceau d'électrons, la cible de réduction du faisceau d'électrons entraînant un ralentissement soudain du faisceau d'électrons et la production de rayons X ; de telle sorte que, sous la contrainte du collimateur de rayons X, les rayons X pointent vers la partition activée du détecteur de comptage de photons.

2. Système d'imagerie par rayonnement selon la revendication 1, dans lequel le collimateur de rayons X est un collimateur à micropore, placé derrière la cible de réduction du faisceau d'électrons.

3. Procédé d'imagerie par rayonnement basé sur le comptage de photons, utilisant le système d'imagerie par rayonnement selon la revendication 1 ou la revendication 2, comprenant les étapes suivantes :

(1) partitionner le détecteur de comptage de photons ;

(2) activer une partition du détecteur de comptage de photons à l'aide du contrôleur de position temporelle ;

(3) la source de rayons X génère des rayons X qui pointent vers la partition activée du détecteur de comptage de photons après avoir été ajustés par le collimateur de rayons X ;

(4) le détecteur de comptage de photons collecte et enregistre les données des partitions ; et

(5) passer à une autre partition du détecteur de comptage de photons, en répétant les étapes (2)-(4), jusqu'à ce que la collecte des données de toutes les partitions du détecteur de comptage de photons soit terminée, et obtenir des images supprimées des rayons diffusés.

4. Procédé d'imagerie par rayonnement selon la revendication 3, dans lequel plus la partition du détecteur de comptage de photons est petite, plus le taux de contribution des rayons diffusés est faible.

FIG. 1

Crystal and wafer bonding

Wafer structure

Pixel electrode binding Pad

Photoelectric conversion crystal material (Si, Se, CZT etc.)

100mm 2000Columns

100mm 2000 Lines

Comparator 1 / Shaper 1
Comparator 2 / Shaper 2
Comparator 3 / Shaper 3
Comparator 4 / Shaper 4

Counter 1
Counter 2
Counter 3
Counter 4

Amplitude discriminator

Bonding Pad

Event pulse amplifier

Pixel structure

2000X2000Pixels Chip

100mm 2000pixels

100mm 2000pixels

Read Out Bus

FIG. 2

FIG. 3

FIG. 4

FIG. 5

Detector

Photon count
chip

Control chip

Substrate

Indium
solder ball

Tin-lead
solder ball

Silicon via

Tin-lead
solder ball

## FIG. 6

## FIG. 7

Pixel array detector

FIG. 8

Display

Filter

X-ray
source

Photon count
detector

Three-dimensional
reconstruction system

Specimen
Scanning stage

R2    R1

Specimen

Filter

X-ray
source

Photon count detector

Scanning stage

FIG. 9

Aluminum,
molybdenum and
rhodium metal filters

Bremsstrahlung
X-ray generator
and X-ray tube

Near monochromatic
light high-energy
radiation

## FIG. 10

Lifting motion

Distance
adjustment

Distance adjustment

Longitudinal
motion

Transverse motion

## FIG. 11

Photon count
detector

R1

R2

X-ray source

Pixel plane
array

Scanning
platform

Specimen

## FIG. 12

Energy threshold 1
sequence

Energy threshold 2
sequence

Energy threshold 3 sequence

Intensity information sequence

Phase contrast sequence

$$\frac{I(x', y', R_2; x_o, y_o, -R_1)}{I_o(x, y, R_2; x_o, y_o, -R_1)} = 1 - \frac{R_2}{M}\nabla^2\varphi_s$$

FIG. 13

Dyed voxel matrix
(digital wax block)

Energy level

FIG. 14

FIG. 15

FIG. 16

FIG. 17

FIG. 18

FIG. 19

FIG. 20

FIG. 21

FIG. 22

Grating

Grating

Diffraction pattern

FIG. 23

Front image

Back image

Absorption image

FIG. 24

Front image

Back image

Refraction angle image

Front image

Back image

FIG. 25

FIG. 26

Scattered ray distribution area

A central ray
projection area
is used as a signal
collection area

FIG. 27

D1

D2

D3

Effective
pixel

Closed
pixel

Pixels that
collect straight
ray signals

Pixels that
collect scattered
ray signals

FIG. 28

FIG. 29

X(N)

X(0) X(1) X(2)

Y(0)
Y(1)
Y(2)

Y(N)

Narrow X-ray beam

Molybdenum or tungsten metal shielding plate

Conventional ray beam

X-ray source

Pencil-shaped thin-tube X-ray collimator

Scanning detector

Effective collection area timing control signal

Timing position controller

Y Direction motion control

X Direction motion control

Partition image data transmission channel

Image collection and splice processor or computer

EP 3 062 093 B1

FIG. 30

FIG. 31

Time-sharing emitted
X-ray beam

Vacuum tube

Electron beam

Electronic gun

Deflection
mechanism

Micropore collimator

Metal tungsten target
or molybdenum target

Scanning detector

FIG. 32

1

FIG. 33

FIG. 34

**EP 3 062 093 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200410053014
- CN 200810166472
- US 2004066904 A1
- CN ZL200910022100